# EUROPEAN PATENT APPLICATION

(11) **EP 3 075 316 A1**
(43) Date of publication of application: **05.10.2016**
(21) Application number: 15161958.2
(22) Date of filing: 31.03.2015
(51) Int. Cl.: A61B 6/00

(54) **Radiation image recording system**

(71) Applicant: Agfa Healthcare, 2640 Mortsel (BE)
(72) Inventor: Exelmans, Walter, 2640 Mortsel (BE); De Broeck, Eric, 2640 Mortsel (BE)
(74) Representative: Verbrugghe, Anne Marie L.

(57) **Abstract**

A system for generating a radiation image of a body with alerting means for alerting an operator when the image recording system is not ready for exposure.

## Description

### FIELD OF THE INVENTION

The present invention relates to a system for exposing a body (patient, animal, object) to penetrating radiation such as x-rays. The system of the present invention has enhanced safety measures.

### BACKGROUND OF THE INVENTION

Nowadays different configurations of systems exist for recording a radiation image such as an x-ray image of a patient, an animal or an object.

Although the description below will focus on radiation imaging of a human, it is clear that the invention likewise is applicable to radiation imaging of an animal in veterinary application or to radiation imaging of an object in cases where the radiation conditions need to be accurately monitored.

Likewise in the description below reference will be made to x-ray imaging. The invention is likewise applicable to imaging by means of other types of penetrating radiation.

In the most conventional configuration a patient who needs to be subjected to radiation imaging is positioned on an examination table in an exposure room provided with an x-ray tube (with x-ray generator) for emitting x-rays.

A radiation detector is positioned under or behind the patient for recording the x-rays passing through the patient's body.

The detector is for example a direct radiography detector which commonly has a receptor layer (a-Si) and a conversion layer (CsI) as well as read out electronics.

Nowadays such detectors are provided with means for wireless transmission of data from the detector to an access point (e.g. via IEEE 802.lln communication protocol).

The detector further has a battery to provide autonomous operation. Although such batteries have a high performance, there is still a moment when the battery needs recharging.

In a control room separated from the x-ray room for the operator's safety, the operator has a workstation or a control console onto which patient identification can be filled in or by means of which patient identification data can be retrieved from a radiology information system. Additionally on this console an examination type can be selected, e.g. by selecting a thumbnail indicative of an examination type and by retrieving associated exposure conditions from memory.

Parameters corresponding with a selected examination type at the console are communicated to the x-ray generator so as to control the generator's settings for the irradiation of the patient.

The operator remains in the control room in the neighbourhood of the workstation or console and gets status feedback from at least one of the x-ray generator and the detector. It is also possible that the operator gets additional feedback about the positioning of the detector and / or the exposure source.

The operator in the control room has an exposure switch coupled to the operator's console.

Prior to exposure the control switch is in IDLE state. Next, when the patient is properly positioned in the exposure room, the operator pushes the switch so that it is in PREP state. In this state exposure source is prepared for exposure (i.e. the rotor of the x-ray system is brought up to speed).

Next, when all exposure conditions are ready, the operator pushes the control button into the EXP state (exposure state) and the exposure is performed.
When the exposure is terminated (after a present exposure time or when an AEC device (Automatic Exposure Control) controls the end of the exposure, the switch goes back to the IDLE state.

In this most conventional embodiment the operator gets feedback via the console in the operator's room and only pushes the switch from the PREP into the EXP position when all conditions are ready for exposure.

Nevertheless it is possible that the operator is distracted and does not pay attention to a warning displayed on the operator's console.

An example of such a warning might be an indication coming from the exposure room that the battery of the direct radiography detector is low implying that the detector is not in a good condition for image recording.

In such situations the operator might push the switch from PREP to EXP without all conditions being optimal. This might lead to an erroneous exposure, in which case the exposure has to be repeated and the patient is a second time subjected to penetrating radiation. This situation is to be avoided.

The situation becomes even be worse in case of another configuration in which a mobile x-ray recording apparatus is used.

For patients who lack the mobility necessary to move to the X-ray room, mobile imaging devices have been developed such as Agfa's DX-D 100. This apparatus comprises an x-ray source and a wireless digital radiography detector. The apparatus has an integrated workstation. When a specific type of exam is selected, the appropriate x-ray settings are automatically transferred to the X-ray generator and displayed on the workstation console. The exposure parameters are added to the digital image file and are communicated seamlessly to PACS, HIS and RIS so that manual data entry and typographical errors are avoided.

A mobile exposure unit of the above-described kind can be either stand alone (non-integrated) or integrated.

In case of a non-integrated embodiment, the user primarily disposes of a detector panel and an exposure unit. The exposure settings are input into the console of the exposure unit. Suchlike units have a hand held exposure button by means of which the effective exposure can be initiated from a position at a distance from the exposure unit itself. In this case, no feedback is provided regarding exposure conditions or panel status.

In case of an integrated embodiment, the user disposes of a panel, an exposure unit and a workstation coupled to the mobile exposure apparatus, the workstation having a user interface for entering patient identification data, selecting an examination type with associated exposure conditions and exposure settings. The workstation is coupled to the exposure source and to the panel and gets feedback on the actual exposure conditions and the panel status.

The workstation is either integrated into the mobile device as a part of the device or is a laptop which is mounted on the mobile device.

Similar as in the non-integrated situation an exposure button (in most cases operating on the basis of wireless communication) is provided allowing the operator to start the effective exposure from a remote position.

In both mobile embodiments the operator handling the exposure button is remote from the workstation or from the console. He protects himself from irradiation and hence preferably stands at a distance from the mobile exposure apparatus. In this position he might even stand in a location in which the mobile exposure apparatus is out of sight.

Hence in these mobile situations it is often impossible for the operator to see the set data or the feedback data on the console or on the monitor. This may lead to dangerous situations.

A cumbersome situation might for example exist in case a self-triggering wireless detector is used. Self-triggering detectors have an internal mechanism for detecting the point in time when the detector initiates the image acquisition state. The initialisation of this state is not triggered by an external signal.
The initialisation self evidently needs to be tuned to the irradiation process.

However, situations may occur in which the operator initiates the exposure by sending a control signal to the x-ray generator to initiate irradiation by the x-ray source and is, because he stands in a safe location remote from the x-ray source, unable to see a warning given by the detector that the detector's battery is low.

With the battery being low, the self-triggering detector will not start the image accumulation process. As the exposure process is already commenced, the patient will be exposed to irradiation without an image being recorded.

This may give rise to necessary re-takes which may be harmful for the patient.

Although this situation has been described in the context of a mobile image acquisition apparatus, it might also occur in case of a fixed, non-mobile embodiment as described higher.

It is thus evident that there is room for improvement of the safety of such systems for recording a radiation image of a patient.

### SUMMARY OF THE INVENTION

The above-mentioned aspects are realised by a system for generating a radiation image of a body having the specific features set out in claim 1.

In the context of this invention the term 'body' may refer to a human patient, an animal or an object.

A system according to the present invention generally has a source of radiation arranged to emit penetrating radiation and a detector for detecting radiation emitted by said source of radiation and modulated said body. Means, e.g. a control button, are provided for activating the operation of said source of radiation.
According to this invention the means for activating operation of said source of radiation are provided with an alert system for generating an alert when at least one of the status of the source of radiation or of the detector is not ready for exposure.

In the context of the present invention the terms 'exposure conditions not met' mean that an exposure cannot be adequately performed (i.e. an exposure would not lead to a valid result). An exposure with suchlike exposure conditions would not lead to the recording of a radiation image or would lead to the recording of a radiation image which is not adequate for performing a diagnosis.

Exposure conditions are not met when the actual situation in the exposure room does not corresponds with the settings required for the envisaged exposure as entered on the operator's console or retrieved from the radiology information system or another data base.
Exposure conditions are not met when e.g. the source of radiation or the detector is not in a state for validly performing the exposure and/or radiation image detection means are not ready to detect the radiation image in a correct way.

Still other components in the radiology room may have an influence on the ready / not ready condition. For example if the supporting table is still moving to the position for exposure, the required conditions for exposure are not fulfilled.

Also the fact that another exposure is already ongoing may be a reason to generate an alert.

Alerts regarding different causes may be identical or different.
For example an alert indicating that an exposure is ongoing may be different from an alert indicating that the detector is not ready to detect the radiation image or from an alert indicating that the relative positioning of the patient supporting table and the detector or the source is not correct to perform a correct patient exposure.

Different alerts may for example have a different auditive frequency or may have different vibrating frequency.

Different embodiments of alerting systems may be envisaged as is set out below.

The alerting system is coupled to the exposure button so that in all cases the operator is alerted at the time when he handles the exposure button.

The alerting system can be arranged to provide an alert and can alternatively or additionally be arranged to disable the operation of the exposure button when the exposure conditions are not ready for exposure or the detector status is not ready for registering a radiation image or when some other conditions in the exposure room are not adapted for the envisaged recording (e.g. table - source position is not correct).

Similarly, the alerting system can be arranged to provide an alert and can be arranged to disable the operation of functions on the workstation or console e.g. preventing a new or change in selection of the selected thumbnail indicative of the examination type for the exposure. This alternative would still allow the prepared exposure to take place, but would prevent the initiation of a next consecutive exposure. This type of alert would prevent the occurence of improperly identified consecutive exposures in a case where e.g. the data communication between the workstation and the detector would not be operational.

In a specific embodiment different alerts can be generated for different conditions that are not met.

Specific features for preferred embodiments of the invention are set out in the dependent claims.

Further advantages and embodiments of the present invention will become apparent from the following description.

### DETAILED DESCRIPTION OF THE INVENTION

A system for generating a radiation image of a patient (or an object or an animal) according to the present invention basically comprises a source of radiation and a radiation detector for detecting the radiation image.

The description below refers to a mobile x-ray imaging system such as Agfa's DX-D 100 apparatus. This embodiment is described by way of example. The present invention is not restricted to such apparatuses and may be implemented in other types of x-ray imaging devices (such as non-mobile x-ray imaging devices).

In the described embodiment the radiation detector is a direct radiography detector which commonly has a receptor layer (a-Si) and a conversion layer (CsI) as well as read out electronics.

The system additionally has a control console for entering patient data and selecting an examination type.

Patient data may be entered manually or may be retrieved from a database such as a radiology information system (RIS) coupled to the console.

Examination type data comprising the type of examination and associated settings for the generator controlling the x-ray source may likewise be entered manually or may be preset and/or retrieved from a database.

The console can be part of the imaging system or may take the form of a (portable) computer communicating (wired or wireless) with the generator and /or source of radiation.

The system further has means for controlling the operation of the source of radiation.
In one embodiment these means comprise a control button coupled to the generator controlling the source of radiation.
The control button can be coupled to the generator either in a wired or wireless way.

In one embodiment the control button is a hand-held device.

According to the invention this control button is provided with means for alerting the operator when the conditions are not adequate to initiate the exposure of the patient.

Several conditions may result in a situation in which the system is not ready for exposure. Examples are incorrect settings of the radiation source, the status of the detector, incompatibilities in the exposure room, other exposure ongoing etc.
An example of an incompatibility in the exposure room is the fact that the positioning of the x-ray source is incompatible with the selected examination type.

The alerting means according to this invention may have different forms.

In one embodiment the alerting means comprise means for generating a visual indication of the status of at least one of the exposure source (and/or generator), the detector or other room or examination conditions.
The alerting means may be a light emitting means provided e.g. on the exposure button.
The visual indication may for example be a green light in case all conditions are ready for exposure, a red light indicating that the there is at least one condition which is not in a state ready for exposure.

In another embodiment the means for generating a visual indication comprise a small display device which is preferably adhered to the exposure button and which is arranged to display data regarding the exposure conditions so that the operator when being remote from the x-ray imaging device and thus also from the console can see on that display at least some of the data that normally are displayed on the console.

In another embodiment the alerting means comprise means for generating an auditive signal.

In still another embodiment the alerting means comprise means that interface in a haptic way with the user, i.e. through the sense of touch. The alerting means may for example be means for generating a vibrating alarm. Most conveniently these means are connected to the exposure button so that the operator who handles the exposure button feels this vibrating alert when he holds the exposure button and is warned by the vibration that exposure conditions are not satisfactory.

In still another embodiment the alerting means are provided on a wearable device such as a smart watch into which the exposure button is integrated or with which the exposure button is coupled, preferably wirelessly coupled.

The exposure button and/or alerting means may be part of a type of wearable technology with an optical head-mounted display such as a smart glass device.

Different alerts may be generated corresponding with different conditions that are not met. These different alerts may be of different types or may be the same type but generating a different signal, such as two vibrating signals with different vibration frequency. Still other embodiments are possible.

In yet another embodiment, the alerting means are provided in the user interface of the console or workstation, where certain functions may be disabled in a case where exposure conditions are not met. An example of such a disabling mechanism is that active screen elements (such as thumbnails or buttons on the screen) may be disabled by visually greying them out, and thus relaying the message to the user that said function is not available or operational at that moment. The selection of said screen elements are then not functional.

In all embodiments it is possible to have an alerting means as described higher.

The alerting means can simply provide an alert to the user that exposure conditions are not satisfactory.

However, it is also possible to design the operation of the alerting means in such a way that upon alert the operation of the device which initiates the exposure (exposure button) is disabled when the alerting means generate a negative alert (exposure conditions not satisfactory for exposure).

## Claims

1. A system for generating a radiation image of a body comprising
- a source of radiation arranged to emit penetrating radiation,
- a detector for detecting radiation emitted by said source of radiation and modulated said body,
- activating means for activating the operation of said source of radiation,
**characterised in that** said activating means is provided with an alert system for generating an alert when an exposure condition is not met.

2. A system according to claim 1 wherein the operation of said means for activating operation of said source of radiation is disabled when an exposure condition is not met.

3. A system according to claim 1 wherein said alert system is adapted to give a visual warning.

4. A system according to claim 1 wherein said alert system comprises a display device giving a visual warning.

5. A system according to claim 1 wherein said alert system is adapted to generate a haptic signal.

6. A system according to claim 5 wherein said haptic signal is a vibration.

7. A system according to claim 1 wherein said alert system is adapted to give an auditive warning.

8. A system according to claim 5, 6 or 7 adapted to generate a number of different warning signals corresponding with different exposure conditions.

9. A system according to claim 8 wherein said different warning signals have different frequencies.

10. A system according to any of claims 1, 5 or 6, 7 or 8 wherein said means for activating the operation of said source of radiation and / or said alerting system are integrated in a wearable device.

11. A system according to claim 1 wherein said means for activating the operation of said source of radiation and / or said alert system are integrated in a smart glass device.

12. A system according to claim 1 wherein said means for activating the operation of said source of radiation is wirelessly coupled to said source of radiation.

13. A system according to claim 1 wherein said exposure source is part of a mobile x-ray imaging unit.

14. A system according to claim 1 wherein said alert system is adapted to generate a visual warning on an operator console or workstation by deactivation of an otherwise active screen element.
